# EUROPEAN PATENT APPLICATION

(11) **EP 4 248 737 A1**
(43) Date of publication of application: **27.09.2023**
(21) Application number: 22163975.0
(22) Date of filing: 24.03.2022
(51) Int. Cl.: A01H 1/00

(54) **OPTIMIZED GENOTYPING AND DATA ANALYTICS ON HAPLOID PLANT MATERIAL**

(71) Applicant: KWS SAAT SE & Co. KGaA, 37574 Einbeck (DE)
(72) Inventor: Ouzunova, Milena, 37077 Göttingen (DE); Knaak, Carsten, 37079 Göttingen (DE); Quintero Prieto-Puga, Javier, 37574 Einbeck (DE); Bolduan, Christof, 37574 Einbeck (DE); Springmann, Clemens, 37581 Bad Gandersheim (DE); Weissleder, Knut, 37574 Einbeck (DE); Mahone, Gregory Stewart, 37574 Einbeck (DE); Oldach, Klaus, 29328 Müden a.d. Örtze (DE)
(74) Representative: Eisenführ Speiser

(57) **Abstract**

Provided is a method for selecting a plant having a desirable genetic constitution or breeding value by evaluating/correlating an estimated breeding value with the genetic constitution of a plant, e.g. for application in plant breeding. The method *inter alia* comprises generating a segregating population from the starting plant, plant material or population, wherein the segregating population comprises one or more individual(s) being haploid; identifying a plant material being haploid with respect to the starting population and monitoring the segregating population, Next, the genetic constitution in a position of the genome in the plant material is determined and optionally evaluated using data analytics methods so that a correlation of a specific genetic constitution with an estimated breeding value takes place. Finally, a desirable genetic constitution or breeding value is identified based on the preceding evaluation. Further provided is a method of producing plants, particularly a doubled haploid plant being homozygous regarding a desired genetic constitution.

## Description

### Technical Field

The present invention provides a method for selecting a plant having a desirable genetic constitution or at least one breeding value based on the evaluation of, or for correlating an estimated breeding value with the genetic constitution of a plant, in particular for application in plant breeding. The method comprises the steps: (i) providing a starting plant, plant material or population of plants; (ii) generating a segregating population from the starting plant, plant material or population of step (i), wherein the segregating population comprises one or more individual(s) being haploid with respect to the population of step (i); (iii) identifying a plant material being haploid with respect to the population of step (i) in one or more individual(s) of the segregating population obtained in step (ii); (iv) determining the genetic constitution in at least one position of the genome in the plant material identified in step (iii), preferably by means of one or more genotyping technique(s); (v) optionally, evaluating the determined genetic constitution using data analytics methods and thus correlating at least one specific genetic constitution with an estimated breeding value; (vi) selecting at least one individual from the segregating population of step (ii) having a desirable genetic constitution as determined in step (iv) or at least one breeding value based on the evaluation of step (v); and (vii) optionally, obtaining at least one plant from the at least one selected individual of step (vi). In the method described above, steps (iii) to (v) are performed on a plant material being haploid with respect to the population of step (i). Further provided is a method of producing a plant, in particular a doubled haploid plant, which is homozygous with respect to a desired genetic constitution, comprising the steps (i) to (vii) as defined in the method of described above.

### Background

The generation and use of haploids is one of the most powerful biotechnological means to improve cultivated plants. The advantage of haploids for breeders is that homozygosity can be achieved already in the first generation after dihaploidization or more generally doubled haploidization by chemical means or by spontaneous chromosome doubling, creating doubled haploid plants, without the need of laborious backcrossing steps to obtain a high degree of homozygosity. Furthermore, the value of haploids in plant research and breeding lies in the fact that the founder cells of doubled haploids are products of meiosis, so that resultant populations constitute pools of diverse recombinant and at the same time genetically fixed individuals. The generation of doubled haploids thus provides perfectly useful genetic variability to select from with regard to crop improvement.

Haploid plants can be obtained by intra- or interspecific crosses, in which one parental genome is eliminated after fertilization. It was shown that genome elimination after fertilization could be induced by modifying a centromere protein, the centromere-specific histone CENH3 in Arabidopsis thaliana (Ravi and Chan, Haploid plants produced by centromere-mediated genome elimination, Nature, Vol. 464, 2010, 615-619). With the modified haploid inducer lines, haploidization occurred in the progeny when a haploid inducer plant was crossed with a wild-type plant. Interestingly, the haploid inducer line was stable upon selfing, suggesting that a competition between modified and wild-type centromere in the developing hybrid embryo results in centromere inactivation of the inducer parent and consequently in uniparental chromosome elimination. Since then, further mutations leading to haploid inducer activity have been identified. Other ways to obtain haploid plants include gynogenesis via ovule- or ovary culture, androgenesis via anther- or microspore culture or embryo rescue in combination with wide hybridization. From a haploid plant, homozygous doubled haploids plant can be obtained by spontaneous chromosome doubling or by application of a chemical compound such as colchicine.

While methods for haploid induction and subsequent chromosome doubling are available, the production of doubled haploids is still often quite inefficient and thus cost and labor intensive.

In order to obtain a doubled haploid plant, which is homozygous with respect to a desired trait for further breeding, it is useful to select suitable haploid material with a desired genetic constitution before chromosome doubling. Thus, production and screening of a large number of doubled haploids, of which many do not have the desired properties, can be avoided.

WO2019/210100A1 discloses a method for obtaining a doubled haploid plant with a selected genotype, wherein the genotype of genetic markers of a haploid embryo in a kernel is determined by analyzing the genotype of endosperm of the kernel. However, the endosperm is triploid, comprising two chromosome sets from one and one chromosome set from the other parent, while the haploid embryo comprises only gametes from the non-haploid induced parent. Therefore, in the method described in WO2019/210100A1, it is necessary to first distinguish between the paternal and maternal chromosomes in the endosperm before the genotype of the haploid embryo can be deduced.

There is still a need for more efficient selection methods for haploid material with a desired genetic constitution for the production of doubled haploid plants in plant breeding.

It was therefore an objective of the present invention to provide a method for the efficient selection of haploid plant material with a desirable genetic constitution for the production of doubled haploid plants, which are homozygous for the genetic constitution.

In particular, the present invention should provide methods, in which breeding values in a haploid material can be correlated with a genetic constitution and thus increase the efficiency of the breeding process.

### Summary of the Invention

In one aspect the present invention relates to a method for selecting a plant having a desirable genetic constitution or at least one breeding value based on the evaluation of, or for correlating at least one estimated breeding value and at least one genetic constitution in at least one individual of a segregating population of plants, in particular for plant breeding, comprising the following steps:
(i) providing a starting plant, plant material or population of plants;
(ii) generating a segregating population from the starting plant, plant material or population of step (i), wherein the segregating population comprises one or more individual(s) being haploid with respect to the population of step (i);
(iii) identifying a plant material being haploid with respect to the population of step (i) in one or more individual(s) of the segregating population obtained in step (ii);
(iv) determining the genetic constitution in at least one position of the genome in the plant material identified in step (iii), preferably by means of one or more genotyping technique(s);
(v) optionally, evaluating the determined genetic constitution using data analytics methods and thus correlating at least one specific genetic constitution with an estimated breeding value;
(vi) selecting at least one individual from the segregating population of step (ii) having a desirable genetic constitution as determined in step (iv) or at least one breeding value based on the evaluation of step (v); and
(vii) optionally, obtaining at least one plant from the at least one selected individual of step (vi);
wherein steps (iii) to (v) are performed on a plant material being haploid with respect to the population of step (i).

In one embodiment of the method described above, steps (iii) to (v) are performed on haploid plant material.

In another embodiment of the method described above, steps (iii) to (v) are performed on diploid plant material.

In one embodiment of the method according to any of the embodiments described above, the starting population in step (i) is produced by a technique selected from the group consisting of crossing, selfing, targeted mutagenesis and random mutagenesis, or a combination of these techniques.

In another embodiment of the method according to any of the embodiments described above, in step (ii) an *in vivo* method using a paternal or maternal haploid inducer or an *in vitro* method using gynogenesis via ovule- or ovary culture, androgenesis via anther- or microspore culture is applied or embryo rescue in combination with wide hybridization is performed.

In one embodiment of the method according to any of the embodiments described above, the plant material in step (iii) is plant material prior to and/or during applying doubling chemicals or spontaneous doubling.

In another embodiment of the method according to any of the embodiments described above, the plant material in step (iii) is a portion of leaves, seeds, seedlings, roots, cells, shoot apex, pollen, flowers, calli or a whole cell.

In a further embodiment of the method according to any of the embodiments described above, the plant material in step (i) is a flower, anther, microspore, ovary or ovula or a part thereof.

In another embodiment of the method according to any of the embodiments described above, the plant material is identified in step (iii) by a method selected from the group consisting of using at least one morphological or visual marker for selection, flow cytometry and chromosome counting. A morphological marker includes, for example, any markerthat can be easily measured and compared, including are usually visual indicators (including visual and measurable on a macroscopic level) of phenotypically differing characters, such as shape, and size of the flower, seeds, or leaves; type of development of plants, inflorescences, or root system; pigmentation; or habit and the like. A visual marker includes, for example, a color or fluorescent marker that has a detectable color phenotype that can be detected with the naked eye, or that can be visualized with the corresponding equipment.

In one embodiment of the method according to any of the embodiments described above, DNA is isolated from the plant material for determining the genetic constitution in step (iv).

In a further embodiment of the method according to any of the embodiments described above, the determination of a genetic constitution comprises or is the determination of a haplotype and/or a marker profile in the plant material. In a certain embodiment, the determination genetic constitution comprises or is the determination of two or more haplotype and/or two or more marker profile in the plant material.

In another embodiment of the method according to any of the embodiments described above, one or more genotyping technique(s) provide(s) at least one genomic information selected from the group consisting of
(a) the nucleotide(s) present or absent at one or more single positions in the genome;
(b) a sequence stretch of nucleotides present or absent in the genome;
(c) fragment length differences of a genomic sequence stretch; and
(d) differences of genomic sequence stretches
is/are applied to determine the genetic constitution in step (iv).

Genomic information preferably refers to information on particular marker alleles at particular loci such as marker loci related to a particular haplotype, or a particular marker profile.

In one embodiment of the method according to any of the embodiments described above, a genotyping technique selected from the group consisting of SNP detection assays, sequencing, tandem repeat assays, INDEL detection assays or probe hybridization assays or a combination thereof is/are applied to determine the genetic constitution in step (iv).

In another embodiment of the method according to any of the embodiments described above, in the evaluation in step (v) the genetic constitution of single or multiple genomic positions is compared to known constitutions to evaluate the presence or absence of a desirable genetic constitution or to estimate a breeding values.

In one embodiment of the method according to any of the embodiments described above, in step (vii) a treatment with chromosome doubling chemicals or spontaneous chromosome doubling takes place.

In another aspect, the present invention relates to a method of producing a plant, which is homozygous with respect to a desired genetic constitution comprising the steps (i) to (vii) as defined in any of the embodiments described above.

In one embodiment of the method of producing a plant described above, the produced plant is a doubled haploid plant.

### Definitions

An "estimated breeding value" in the context of the present invention is defined as an organism's genetic merit for a particular phenotypic trait (i.e., phenotypic property). Estimated breeding values or EBVs are measurements of heritable phenotypic traits. EBVs can be predicted for a plurality of plant individuals e.g. for improving breeding decisions. EBVs can for example be calculated by using specialized computer programs, such as ASReml or Residual Maximum Likelihood (REML) techniques in an R environment. These programs can account and may in some cases adjust phenotypic observations to other known impact factors such as sex, growing season, plant age, soil type, etc. An estimated breeding value thus allows to define a desirable genetic constitution at one or several positions of the genome and thus to establish a link between a genotype and a phenotype, the phenotype having favourable characteristics in breeding.

The term "genetic constitution" in the context of the present invention relates to one or a combination of genetic feature(s) in one or more positions of the genome of an individual. Such features may be determined by genotyping methods. For example, the genetic constitution of a plant can be specified by the presence or absence of certain nucleotides at one or more positions in the genome, by the presence or absence of a sequence stretch in the genome, by the fragment length of a sequence stretch present in the genome or a mixture of the above. Alternatively or additionally, the genetic constitution can also mean a particular haplotype or a particular marker profile. Preferably, the haplotype or marker profile is within, linked to or associated with one or more QTLs associated with a particular trait or a particular germplasm or a particular plant. The haplotype or marker profile can comprise two or more polymorphisms, i.e. marker loci. In some embodiments, the haplotype or the marker profile may comprise alleles favorable for a particular trait, a particular germplasm or a particular plant.

"Haplotype" refers to a combination of particular alleles present within a particular plant's genome at two or more linked marker loci, for instance at two or more loci on a particular linkage group. For instance, in one embodiment, two specific marker loci on a particular chromosome are used to define a haplotype for a particular plant. In still further embodiments, 3, 4, 5, 6, 7, 8, 9, 10, 11, 12, 13, 14, 15, 16, 17, 18, 19, 20, or more linked marker loci are used to define a haplotype for a particular trait, a particular germplasm or a particular plant.

As used herein, a "marker profile" means a combination of particular alleles present within a particular plant's genome at two or more marker loci which are not linked, for instance two or more loci on two or more different linkage groups or two or more chromosomes. For instance, in one embodiment, one marker locus on a particular chromosome and a marker locus on another linkage group are used to define a marker profile for a particular germplasm or a particular plant. In certain other embodiments a plant's marker profile comprises one or more haplotypes. In some embodiments, the marker profile encompasses two or more loci for the same trait, such as dry matter yield, drought tolerance, pathogen resistance or flowering time. In other embodiments, the marker profile encompasses two or more loci associated with two or more traits of interest.

"Genotyping method" or "Genotyping technique" refers to a method, which allows to detect specific features in a genome, e.g. single nucleotide polymorphisms (SNPs), the presence or absence of sequence stretches, insertions or deletions of one or more nucleotides or fragments lengths polymorphisms. Available methods include SNP detection assays, sequencing, tandem repeat assays, INDEL detections assays and probe hybridization assays.

A "segregating population" is a population of individuals which is genetically divers, i.e. genetic differences can be observed between the individuals. A segregating population may be the progeny resulting from a cross of parents, which differ in one or more traits, or from selfing of a heterozygous parent, which differ in one or more traits, or of a targeted or random mutagenesis. A targeted mutagenesis results e.g. in a sequence variation in a specific location of the genome of an individual. Site-specific nucleases may e.g. be used to introduce a double strand break in a specific location, which may then be repaired by different mechanisms resulting in an altered genomic sequence. Random mutagenesis, on the other hand, is performed by the application of e.g. mutagenic chemicals, transposable elements or UV radiation and results in random mutations in the genome.

As used herein, the term "haploid plant" means a plant having a single set (genome) of chromosomes and the reduced number of chromosomes (n) in the haploid plant is equal to that in the gamete. As used herein, the term "diploid plant" means a plant having two sets (genomes) of chromosomes and the chromosome number (2n) is equal to that in the zygote. As used herein, the term "doubled haploid" or "doubled haploid plant" or "doubled haploid cell" means one that is developed by the doubling of a haploid set of chromosomes. A plant or seed that is obtained from a doubled haploid plant that is selfed any number of generations is still identified as a doubled haploid plant. A doubled haploid plant is considered a homozygous plant. A plant is considered a doubled haploid if it is fertile, even if the entire vegetative part of the plant does not consist of the cells with the doubled set of chromosomes. For example, a plant will be considered a doubled haploid plant if it contains viable gametes, even if the plant is chimeric.

Doubled haploid in general can refer to a material sampled and tested "on haploid level", where a chromosome doubling has been performed.

"Haploid with respect to the population of step (i)" in the context of the present invention means that at least one individual in the population of step (ii) has only half the ploidy of the starting population in step (i). For example, if the starting population is diploid, at least one individual generated in step (ii) is haploid, or, if the starting population is tetraploid, at least one individual generated in step (ii) is diploid etc. Thus, in step (ii) haploidization takes place, which produces at least one individual with half the ploidy of the population in step (i).

"Data analytics methods" refer to methods, which use the determined genetic constitution of a plant material to compare the given constitution to known constitutions (reference constitutions). Such comparisons are the estimation of the presence or absence of a desired genetic constitution of one or the combination of single genetic constitutions, e.g. one or multiple stretches of multiple nucleotide positions, the presence or absence of one or multiple insertions/deletions or desired fragment length polymorphisms or hybridization of known probes. The methods also cover the estimation of breeding values by using a precalculated relation between genetic constitutions and a plant characteristic to estimate the breeding value of such plants by applying the known relation to the constitutions of new plant material. Such pre-calculated relation between genetic constitution and plant characteristics are obtained by models using recombination theory like quantitative trait loci (QTL) statistics, methods for genome wide association or mixed models, Bayesian and machine learning algorithms as used in genomic prediction.

"Doubling chemicals" are chemical compounds, which - when applied to haploid plants - facilitate the doubling of the chromosomes resulting in at least some doubled haploid plant. The most commonly used doubling chemical is colchicine. "Spontaneous doubling", on the other hand, refers to chromosome doubling, which occurs spontaneously in some cases without intervention.

A plant is "homozygous with respect to a desired genetic constitution", when the genomic features defining the desired genetic constitution are present on both, or respectively, all homologous chromosomes. Further, a plant "homozygous with respect to a desired genetic constitution" can be obtained from a plant cell, tissue, organ, seed, seedling, plant callus or any another plant from which a viable plant can be obtained.

### Detailed Description

The present invention provides efficient methods, which apply genotyping and data analytics on haploid plant material prior to or during chromosome doubling and development of diploid homozygous plants. By genotyping on haploid level it is possible to increase significantly the efficiency of the breeding process through increase of the selection intensity and enhancement of the selection gain.

In one aspect, the present invention relates to a method for selecting a plant having a desirable genetic constitution or at least one breeding value based on the evaluation of, or for correlating at least one estimated breeding value and at least one genetic constitution in at least one individual of a segregating population of plants, in particular for plant breeding, comprising the following steps:
(i) providing a starting plant, plant material or population of plants;
(ii) generating a segregating population from the starting plant, plant material or population of step (i), wherein the segregating population comprises one or more individual(s) being haploid with respect to the population of step (i);
(iii) identifying a plant material being haploid with respect to the population of step (i) in one or more individual(s) of the segregating population obtained in step (ii);
(iv) determining the genetic constitution in at least one position of the genome in the plant material identified in step (iii), preferably by means of one or more genotyping technique(s);
(v) optionally, evaluating the determined genetic constitution using data analytics methods and thus correlating at least one specific genetic constitution with an estimated breeding value;
(vi) selecting at least one individual from the segregating population of step (ii) having a desirable genetic constitution as determined in step (iv) or at least one breeding value based on the evaluation of step (v); and
(vii) optionally, obtaining at least one plant from the at least one selected individual of step (vi).

Notably, steps (iii) to (v) are performed on a plant material being haploid with respect to the population of step (i).

Using the method according to the invention, it is possible to select the most valuable plants for further breeding at the haploid stage and thus avoid production and screening of a large number of doubled haploid plants, of which many will not have the desired genetic constitution and thus not provide a significant value for further breeding.

Genetic features are analyzed by genotyping techniques to determine the genetic constitution in at least one position and the determined genetic constitution is then compared to (a) known genetic constitution(s) by data analytics methods to determine an estimated breeding value. Thus, a haploid individual can be identified, which will likely impact the genome of the progeny in a desirable way when the individual is used for breeding after chromosome doubling.

In one embodiment of the method described above, step (vii) comprises chromosome doubling either by applying a doubling chemical, such as colchicine, or by spontaneous chromosome doubling.

In a preferred embodiment of the method described above, steps (iii) to (v) are performed on haploid plant material.

In this case, the starting population provided in step (i) is diploid and in step (ii) at least one haploid individual is generated by haploidization. By chromosome doubling, a homozygous doubled haploid plant is generated in step (vii) from the selected haploid material having a desired genetic constitution.

In another preferred embodiment of the method described above, steps (iii) to (v) are performed on diploid plant material.

In this case, the starting population provided in step (i) is tetraploid and in step (ii) at least one diploid individual is generated by haploidization. By chromosome doubling, a tetraploid haploid plant may be generated in step (vii) from the selected diploid material having a desired genetic constitution or a plant can be directly obtained from the diploid material.

The starting population of step (i) can be provided by a number of methods but is preferably a segregating population providing some genetic diversity from which a desirable genetic constitution can be selected.

In one embodiment of the method according to any of the embodiments described above, the starting population in step (i) is produced by a technique selected from the group consisting of crossing, selfing, targeted mutagenesis and random mutagenesis, or a combination of these techniques.

In step (ii), a haploidization step takes place, which provides at least one individual having half the ploidy of the starting population. The skilled person is aware of techniques to generate haploid plants.

In one embodiment of the method according to any of the embodiments described above, in step (ii) an *in vivo* method using a paternal or maternal haploid inducer or an *in vitro* method using gynogenesis via ovule or ovary culture, androgenesis via anther or microspore culture is applied or embryo rescue in combination with wide hybridization is performed.

A haploid plant develops from haploid cells from an unfertilized ovule (gynogenesis), or haploid cells from anthers (androgenesis). The frequency of natural haploids is fairly low, about 1 per 1000 in the case of parthenogenesis and about 0.1 per 1000 in the case of androgenesis. Because of the low efficiency of natural occurring haploids, *in vitro* tissue culture methods have been worked out over the years to provide plant breeders with sufficient numbers of doubled haploids in order to partially or completely replace inbreeding.

Anther and microspore cultures are well established techniques which are used for the production of homozygous lines in many crop species, such as maize (Zea mays L.): Gaillard et al. Plant Cell Reports: 10: 55-58 (1991), rice (Oryza sativa L.): Raina et al. Plant Cell Reports: 6: 43-45, (1987), oilseed rape (Brassica napus): Keller W. and Armstrong K. Z. Pflanzenzuchting 80, 100-108 (1978), barley (Hordeum vulgare L.): Ziauddin et al. Plant Cell Reports 9: 69-72 (1990), egg-plant (Solanum melongena L.): Tuberosa R. et al. Genet. Agr. 41; 267-274 (1987), broccoli (Brassica oleracea var. Italica): Takahata Y. and Keller W. Plant Science, 74, 235-242 (1991), safflower (Carthamus tinctorius L.): Plant Cell Reports 10: 48-51 (1991), asparagus (Asparagus officinalis) Pelletier G. et al. C.R. Ac. Sci. Paris. Ser. D 274, 848-851 (1972).

Further, certain *in vivo* haploid induction techniques are meanwhile available to the skilled person (see Kalinowska et al., Theor Appl Genet. 2019; 132(3): 593-605).

Haploids and doubled haploids can also be derived from gametophytic cells of the ovary in barley (Hordeum vulgare L.) (San Noeum L. (Ann. Amelior. Plantes 26, 751-754 (1976)). Doubled haploid production via ovary cells is suitable for crop species that are in many cases not amenable for anther or microspore culture. Examples are sunflower (Helianthus annuus L.) Gelebart P. and San L. Agronomie, 7, 81-86 (1987), sugar beet (Beta vulgaris L.) Hosemans D and Bossoutrot D. Z. Pflanzenzucht 91: 74-77 (1983), melon (Cucumis melo L.) Cuny et al. Agronomie, 12, 623-630 (1992), watermelon (Citrullus lanatus (Thunb.)) Sari N et al. Scientia Horticulturae 82, 265-277 (1999), cucumber (Cucumis sativus L.) Dirks R. U.S. Pat. No. 5,492,827 (1995).

The plant material in step (iii) can be identified before chromosome doubling or even during the application of doubling chemicals or during spontaneous chromosome doubling.

In one embodiment of the method according to any of the embodiments described above, the plant material in step (iii) is plant material prior to and/or during applying doubling chemicals or spontaneous doubling.

The plant material identified in step (iii), may be a plant tissue from different parts of the plant or maybe a single plant cell.

In one embodiment of the method according to any of the embodiments described above, the plant material in step (iii) is a portion of leaves, seeds, seedlings, roots, cells, shoot apex, pollen, flowers, calli or a whole cell.

In a further embodiment of the method according to any of the embodiments described above, the plant material in step (i) is a flower, anther, microspore, ovary or ovula or a part thereof.

For determining the ploidy of the plant material, several methods are known to the skilled person. For example, morphological or visual markers, flow cytometry or chromosome counting may be used.

In one embodiment of the method according to any of the embodiments described above, the plant material is identified in step (iii) by a method selected from the group consisting of using at least one morphological or visible marker for selection, flow cytometry and chromosome counting.

In order to determine the genetic constitution in at least one position of the genome in the plant material identified in step (iii), DNA can be isolated to perform one or more assays as required.

In one embodiment of the method according to any of the embodiments described above, DNA is isolated from the plant material for determining the genetic constitution in step (iv).

By performing different assays on the DNA isolated from the plant material, information becomes available, which allows to determine the genetic constitution in at least one position of the genome in the plant material. Such information may relate to the genome sequence in one or more locations or provide insight in polymorphisms.

In one embodiment of the method according to any of the embodiments described above, one or more genotyping technique(s) provide(s) at least one genomic information selected from the group consisting of
(a) the nucleotide(s) present or absent at one or more single positions in the genome;
(b) a sequence stretch of nucleotides present or absent in the genome;
(c) fragment length differences of a genomic sequence stretch; and
(d) differences of genomic sequence stretches
is/are applied to determine the genetic constitution in step (iv).

In order to gain such information, different genotyping methods are available.

In one embodiment of the method according to any of the embodiments described above, a genotyping technique selected from the group consisting of SNP detection assays, sequencing, tandem repeat assays, INDEL detection assays or probe hybridization assays or a combination thereof is/are applied to determine the genetic constitution in step (iv).

In a further embodiment, once the genetic constitution in at least one position of the genome is determined, the genetic constitution is evaluated using data analytics methods. In these methods, the determined genetic constitution is compared to known constitutions and based on this comparison correlated with an estimated breeding value. This method allows to select an individual with a desirable genetic constitution for application such as e.g., genomic prediction, marker assisted backcrossing or the presence or absence of certain haplotypes/alleles at one or more positions in the genome for applications such as e.g. marker assisted selection in forward breeding or in trait integration.

In one embodiment of the method according to any of the embodiments described above, in the evaluation in step (v) the genetic constitution of single or multiple genomic positions is compared to known constitutions to evaluate the presence or absence of a desirable genetic constitution or to estimate a breeding values.

Obtaining a plant for further breeding application in step (vii) may comprise the use of doubling chemicals or spontaneous chromosome doubling.

In one embodiment of the method according to any of the embodiments described above, in step (vii) a treatment with chromosome doubling chemicals or spontaneous chromosome doubling takes place.

In a further aspect, the present invention relates to a method of producing a plant, which is homozygous with respect to a desired genetic constitution comprising the steps (i) to (vii) as defined in any of the embodiments described above.

In a preferred embodiment of the method of producing a plant described above, the produced plant is a doubled haploid plant.

In this case, the starting population provided in step (i) is diploid and in step (ii) at least one haploid individual is generated by haploidization. By chromosome doubling, a homozygous doubled haploid plant is generated in step (vii) from the selected haploid material having a desired genetic constitution.

## Claims

1. A method for selecting a plant having a desirable genetic constitution or at least one breeding value based on the evaluation of, or for correlating at least one estimated breeding value and at least one genetic constitution in at least one individual of a segregating population of plants, in particular for plant breeding, comprising the following steps:
(i) providing a starting plant, plant material or population of plants;
(ii) generating a segregating population from the starting plant, plant material or population of step (i), wherein the segregating population comprises one or more individual(s) being haploid with respect to the population of step (i);
(iii) identifying a plant material being haploid with respect to the population of step (i) in one or more individual(s) of the segregating population obtained in step (ii);
(iv) determining the genetic constitution in at least one position of the genome in the plant material identified in step (iii), preferably by means of one or more genotyping technique(s);
(v) optionally, evaluating the determined genetic constitution using data analytics methods and thus correlating at least one specific genetic constitution with an estimated breeding value;
(vi) selecting at least one individual from the segregating population of step (ii) having a desirable genetic constitution as determined in step (iv) or at least one breeding value based on the evaluation of step (v); and
(vii) optionally, obtaining at least one plant from the at least one selected individual of step (vi);
wherein steps (iii) to (v) are performed on a plant material being haploid with respect to the population of step (i).

2. The method according to claim 1, wherein steps (iii) to (v) are performed on haploid plant material.

3. The method according to any of the preceding claims, wherein the starting population in step (i) is produced by a technique selected from the group consisting of targeted mutagenesis and random mutagenesis, or a combination of these techniques.

4. The method according to any of the preceding claims, wherein in step (ii) an *in vivo* method using a paternal or maternal haploid inducer or an *in vitro* method using gynogenesis via ovule or ovary culture, androgenesis via anther or microspore culture is applied or embryo rescue in combination with wide hybridization is performed.

5. The method according to any of the preceding claims, wherein the plant material in step (iii) is plant material prior to and/or during applying doubling chemicals or spontaneous doubling.

6. The method according to any of the preceding claims, wherein the plant material in step (iii) is a portion of leaves, seeds, seedlings, roots, cells, shoot apex, pollen, flowers, calli or a whole cell.

7. The method according to any of the preceding claims, wherein the plant material is identified in step (iii) by a method selected from the group consisting of using at least one morphological or visual marker for selection, flow cytometry and chromosome counting.

8. The method according to any of the preceding claims, wherein DNA is isolated from the plant material for determining the genetic constitution in step (iv).

9. The method according to any of the preceding claims, wherein determining the genetic constitution in step (iv) comprises or is the determination of a haplotype and/or a marker profile in the plant material.

10. The method according to any of the preceding claims, wherein one or more genotyping technique(s) provide(s) at least one genomic information selected from the group consisting of
(a) the nucleotide(s) present or absent at one or more single positions in the genome;
(b) a sequence stretch of nucleotides present or absent in the genome;
(c) fragment length differences of a genomic sequence stretch; and
(d) differences of genomic sequence stretches
is/are applied to determine the genetic constitution in step (iv).

11. The method according to any of the preceding claims, wherein a genotyping technique selected from the group consisting of SNP detection assays, sequencing, tandem repeat assays, INDEL detection assays or probe hybridization assays or a combination thereof is/are applied to determine the genetic constitution in step (iv).

12. The method according to any of the preceding claims, wherein in the evaluation in step (v) the genetic constitution of single or multiple genomic positions is compared to known constitutions to evaluate the presence or absence of a desirable genetic constitution or to estimate a breeding values.

13. The method according to any of the preceding claims, wherein in step (vii) a treatment with chromosome doubling chemicals or spontaneous chromosome doubling takes place.

14. A method of producing a plant, which is homozygous with respect to a desired genetic constitution comprising the steps (i) to (vii) as defined in any of claims 1 to 13.

15. The method according to claim 14, wherein the produced plant is a doubled haploid plant.
